**Europäisches Patentamt**

**European Patent Office**

**Office européen des brevets**

(19)

(11) Numéro de publication : **0 452 598 B1**

# FASCICULE DE BREVET EUROPEEN

(12)

(45) Date de publication du fascicule du brevet :
**24.11.93 Bulletin 93/47**

(51) Int. Cl.[5] : **A61K 37/02**

(21) Numéro de dépôt : **90401533.6**

(22) Date de dépôt : **06.06.90**

(54) **Utilisation d'un polypeptide ayant l'activité de l'interleukine 2 humaine pour préparer une composition pharmaceutique destinée au traitement de cancers primitifs de la plèvre.**

(30) Priorité : **17.04.90 FR 9004895**

(43) Date de publication de la demande :
**23.10.91 Bulletin 91/43**

(45) Mention de la délivrance du brevet :
**24.11.93 Bulletin 93/47**

(84) Etats contractants désignés :
**AT BE CH DE DK FR GB IT LI LU NL SE**

(56) Documents cités :
**EP-A- 0 353 150**
**PROCEEDINGS OF THE AMERICAN ASSOCIA-
TION FOR CANCER RESEARCH, vol.31, mars
1990, page 275, résumé no. 1630; G.STOTER et
al.: "Intrapleural interleukin-2 (IL-2) in malignant pleural mesothelioma: A phase I-II study"**
**PROCEEDINGS OF THE AMERICAN ASSOCIA-
TION FOR CANCER RESEARCH, vol. 31, mars
1990, page 274, résumé no. 1622; J.-C. LOU-
RANT et al.: "Cytotoxic response of blood
lymphocytus from patients with meligament
pleurel effusions treated with intro pleural
introleukin-2"**

(56) Documents cités :
**NATURAL IMMUNITY AND CELL GROWTH
REGULATION, vol. 9, no.2, mars-avril 1990,
page 74; A.M.M. EGGERMONT et al.:
"Intrapleural administration of recombinant
interleukin-2 (IL.2) in patients with malignant
pleural mesothelioma: A phase I-II study"**
**CANCER TREATMENT REVIEWS, vol. 16,
suppl. A, 1989, pages 161-162, Academic
Press Ltd; N. THATCHER et al.: "Interleukin-2
in malignant pleural mesothelioma (and adenocarcinoma of the lung). The use of intrapleural and continuous intravenous infusions:
preliminary results"**
**REV. PHNEUMOL. CLIN., vol. 46, no. 5, 1990; C.
BOUTIN et al.: "Le traitement des mésothéliomes par interféron gamma et interleukine
2"**

(73) Titulaire : **ROUSSEL-UCLAF**
**35, Boulevard des Invalides**
**F-75007 Paris (FR)**

(72) Inventeur : **Brandely, Maud**
**135 Boulevard Malesherbes**
**F-75017 Paris (FR)**
Inventeur : **Lando, Danielle**
**17-19, rue de la Plaine**
**F-75020 Paris (FR)**

(74) Mandataire : **Vieillefosse, Jean-Claude et al**
**ROUSSEL UCLAF Département des Brevets**
**111, Route de Noisy B.P. 9**
**F-93230 Romainville (FR)**

## Description

La présente invention concerne l'utilisation d'un polypeptide ayant l'activité de l'interleukine 2 humaine pour la préparation de compositions pharmaceutiques destinées au traitement de cancers primitifs de la plèvre.

L'IL2 qui est une lymphokine produite par les lymphocytes T activés possède une activité immunomodulatrice et une activité antitumorale décrites par exemple par Fletcher, M et al. (Lymphokine Research 6 1987 47-57), activités qui comprennent en particulier la capacité d'initier la prolifération des lymphocytes T et l'induction de la cytotoxicité des cellules NK (natural killer) et des cellules LAK (lymphokine activated killer). On a observé que l'administration de l'IL2 soit seule à haute dose soit associée avec des cellules LAK est capable d'induire la régression de certains cancers établis chez la souris et chez des patients ayant des cancers métastatiques tel que le mélanome, le cancer du rein, le cancer colorectal ou le lymphome non Hodgkinien (Rosenberg,S A et al. N. Eng. J. Med. 1987 316 889-897).

Les cancers primitifs de la plèvre comprennent essentiellement les mésothéliomes diffus et plus rarement les sarcomes pour lesquels les traitements existants de chirurgie, de chimiothérapie ou de radiothérapie seuls ou associés entre eux ne présentent pas d'efficacité reconnue. Ainsi le mésothéliome malin dont l'association avec l'exposition à l'amiante a été suggérée puis confirmée (Antman, KH N. Eng. J. Med. 1980 303 200-202) dans 10 à 70 % des cas (Antman, KH et al. Am. J. Med. 1980 68 356-362) et localisé dans la plèvre, de façon plus fréquente que dans le péritoine (rapport environ 2,5/1), a une évolution mortelle en quelques mois, en l'absence de traitement efficace.

Il a été montré in vitro sur des lignées cellulaires ou sur des cellules fraiches de mésothéliome malin humain que les cellules de mésothéliome sont résistantes (> 99,9 %) à la lyse par les cellules NK, mais relativement sensibles (58 %) à la lyse par des cellules LAK (Manning L S et al. Am. Rev. Respir. Dis June 1989 139 1369-1374).

Des études cliniques ont été conduites, notamment avec un analogue de l'IL2 associé avec une immunothérapie adoptive chez des patients porteurs de tumeurs variées. Une activité antitumorale objective n'a été rapportée que dans le cancer du rein, le mélanome, le cancer du colon et le lymphome. Le mésothéliome est cité dans la liste des tumeurs étudiées qui auraient présenté une régression (Bradley E C et al. Third International Conference on Malignant Lymphoma June 10-13 Lugano 1987 p. 26).

Aucun résultat clinique n'a montré l'efficacité de la thérapie dans les cancers primitifs de la plèvre avec l'IL2 seule, notamment chez 3 patients atteints de mésothéliome ayant reçu une administration par voie intraveineuse (Thatcher N et al. Cancer Treatement Reviews 16 Suppl A 161-162 June 1989).

Or la demanderesse vient d'obtenir des résultats montrant que l'IL2 présente une activité sur les cancers de la plèvre.

L'invention concerne donc l'utilisation d'un polypeptide ayant l'activité de l'interleukine 2 humaine pour préparer une composition pharmaceutique destinée au traitement de cancers primitifs de la plèvre.

On entend par polypeptide ayant l'activité de l'IL2 humaine, l'IL2 humaine naturelle, l'IL2 humaine recombinante, c'est-à-dire obtenue par la technologie de l'ADN recombinant, par exemple comme cela est décrit par Taniguchi, T. et al. (Nature (1983) 302 305-310) ou dans le brevet EP 91539 B, des allèles ou des dérivés de ces produits tels que décrits par exemple par Ju, C. et al. (J. Biol. Chem. (1987) 262 5723-5731).

Les cancers primitifs de la plèvre que concerne l'inven- tion comprennent des cancers tel que le mésothéliome pleural et le sarcome, généralement caractérisés cliniquement par un épanchement pleural.

L'invention a notamment pour objet l'utilisation caractérisée en ce que le cancer de la plèvre est un mésothéliome.

Le mésothéliome pleural est généralement caractérisé cliniquement par des douleurs de la poitrine ou des difficultés respiratoires associées à un épanchement pleural et son diagnostic le différencie de la pleurésie inflammatoire, du cancer primitif du poumon ou de métastases provenant d'un autre cancer primitif. L'invention décrit l'utilisation d'IL2 humaine dans un traitement dont l'efficacité est montrée par un taux de réponse d'environ 50 % chez des malades atteints de mésothéliome pleural, ayant éventuellement subi une chirurgie d'exérèse mais pas de traitement antérieur de chimiothérapie ou de radiothérapie.

L'invention a particulièrement pour objet l'utilisation caractérisée en ce que le polypeptide ayant l'activité de l'IL2 humaine est une IL2 recombinante pure.

Les compositions pharmaceutiques préparées selon l'invention renferment une IL2 humaine recombinante, des allèles ou des dérivés de celle-ci, telle que décrite ci-dessus, pour laquelle on utilise des techniques de purification connues de l'homme de métier, qui permettent de préparer des produits purs.

L'invention a plus particulièrement pour objet l'utilisation caractérisée en ce que l'IL2 est une IL2 recombinante non glycosylée sous forme réduite. L'IL2 non glycosylée utilisée est notamment celle ayant la séquence de l'IL2 naturelle à 133 aminoacides avec éventuellement une Méthionine N-terminale supplémentaire, dont les 3 cystéines en position 58, 105 et 125 sont sous la forme réduite, manifestant une activité biologique comparable à celle de l'IL2 oxydée ayant la même séquence comportant un pont disulfure en position 58-105. L'IL2 réduite est décrite dans la demande de brevet européen EP 0353150. Par forme réduite, on entend que les restes cystéines que contient l'IL2 renferment un groupement sulfhydryle libre dont la détermination est faite, par exemple, par spectrophotométrie avec la dithiodipyridine comme réactif des thiols. L'activité biologique est déterminée par mesure de la prolifération de lignées cellulaires leucémiques de souris dépendantes de l'IL2, CTLL-2, avec un test colorimétrique au sel de tétrazolium (Mossmann, T. J. Immunol. Meth (1983) $\underline{65}$ 55-63). L'activité spécifique des IL2 recombinantes utilisées dans l'invention est au moins égale à $0,5 \times 10^6$ U BRMP/mg, de préférence au moins égale à $1 \times 10^7$ U BRMP/mg. L'unité d'activité IL2 est définie comme la quantité qui produit 50 % de la réponse maximum dans le test. On utilise comme standard un échantillon "Biological Response Modifier Programm (BRMP) reference agent human IL2 (jurkat)" fourni par le National Cancer Institute (NCI).

L'invention a spécialement pour objet l'utilisation caractérisée en ce que l'IL2 est administrée par voie intrapleurale à la dose de 2 à $25 \times 10^6$ $U/M^2$ par injection, et plus spécialement celle caractérisée en ce que l'IL2 est administrée à la dose de $15.10^6$ $U/M^2$ par jour.

L'invention a tout spécialement pour objet l'utilisation caractérisée en ce que l'IL2 est administrée par cycle de 5 jours consécutifs et caractérisée en ce que l'IL2 est administrée de façon répétée pendant au moins deux cycles non consécutifs.

La dose administrée, la fréquence de l'injection et la durée du traitement varient en fonction de l'état du malade.

L'IL2 est comprise dans une composition pharmaceutique, de préférence lyophilisée en flacon-dose contenant de 0,1 à 2 mg de principe actif et que l'on reconstitue avec de l'eau distillée pour injection. La solution obtenue est immédiatement diluée à l'aide d'un soluté, par exemple le glucose à 5 % pour l'administration en perfusion intrapleurale.

Selon l'utilisation préférée de l'invention, l'IL2 est l'IL2 recombinante réduite ci-dessus dont un exemple de préparation pharmaceutique est donnée plus loin, la dose est de $15 \times 10^6$ $U/M^2$ par jour, selon un cycle de 5 jours consécutifs répété 2 fois de façon espacée sur une période d'environ 5 semaines, représentant environ $225 \times 10^6$ $U/M^2$ et environ 21 mg d'IL2 administrés au total chez le malade en perfusion continue par voie intrapleurale.

Les exemples suivants illustrent l'invention sans toutefois la limiter.

### Exemple 1 : Composition pharmaceutique pour perfusion.

On a réalisé une préparation pour injection par voie intraveineuse en perfusion de formule :

| | |
|---|---|
| IL2 réduite | 0,5 mg |
| acide citrique | 5 mg |
| mannitol | 50 mg |
| eau stérile | 1 cm³ |
| glucose à 5 % | 50 cm³ |

### Exemple 2 : Etude clinique dans le traitement de mésothéliome pleural.

L'étude inclut des malades ayant un mésothéliome pleural histologiquement confirmé, confiné à la plèvre, éventuellement étendu au poumon, au thorax ou aux ganglions lymphatiques (stades I, II, III ou IV selon la classification de Butchart) et n'ayant eu aucun traitement antérieur par chimiothérapie ou radiothérapie.

Les malades peuvent avoir éventuellement subi un traitement par chirurgie d'exérèse et présenter une récurrence de la maladie.

Les compositions d'IL2 préparées selon l'invention permettent d'injecter des doses de 3 à $24 \times 10^6$ $U/M^2$ par jour, selon le malade, en perfusion continue par voie intrapleurale pendant un cycle de 5 jours pendant la première semaine, répété pendant la troisième semaine, puis la cinquième semaine du traitement.

Les malades qui présentent une rechute ultérieure ou seulement soit une réponse partielle soit une stabilisation de la maladie sont à nouveau traités par voie intrapleurale comme décrit ci-dessus.

Les lésions tumorales des malades sont évaluées avant et à la fin du traitement par la mesure des lésions macroscopiques au scanner thoracique et à la thorascopie. Une confirmation histologique est obtenue par biopsies multiples des zones précédemment envahies et biopsies au hasard des plèvres pariétale et diaphragmatique.

Sur 11 malades réévalués, les réponses suivantes ont été obtenues :

| PATIENT | Age (ans) | Dose d'IL2 (R4 49637) ($10^6$ U/m2/jour) | Stade | Réponse |
|---------|-----------|------------------------------------------|-------|---------|
| MOU | 64 | 3 | I | RC |
| CAS | 64 | 3 | III | Pg |
| GER | 60 | 6 | II | Pg |
| RAG | 50 | 10 | I | RC |
| CHI | 60 | 10 | III | Pg |
| CHA | 65 | 10 | IV | Pg |
| LLI | 42 | 15 | $I_A$ | RC |
| REY | 59 | 21 | II | Pg |
| SEB | 62 | 21 | I | RP |
| POI | 51 | 21 | II | RP |
| BER | 59 | 24 | II | RC |

Les résultats montrent 4 réponses complètes (RC) et 2 réponses partielles (RP), soit un taux de réponse d'environ 50 %. Une progression (Pg) de la maladie n'est observée que chez des malades de stade II à IV.

**Revendications**

1. Utilisation d'un polypeptide ayant l'activité de l'interleukine 2 humaine pour préparer une composition pharmaceutique destinée au traitement de cancers primitifs de la plèvre, par administration par voie intrapleurale.

2. Utilisation selon la revendication 1 caractérisée en ce que le cancer de la plèvre est un mésothéliome.

3. Utilisation selon la revendication 2 caractérisée en ce que le polypeptide ayant l'activité de l'IL2 humaine est une IL2 recombinante pure.

4. Utilisation selon la revendication 3 caractérisée en ce que l'IL2 est une IL2 recombinante non glycosylée sous forme réduite.

5. Utilisation selon la revendication 3 ou 4 caractérisée en ce que l'IL2 est comprise dans une composition pharmaceutique qui permet l'administration de l'IL2 par voie intrapleurale à la dose de 2 à 25 x $10^6$ U/M$^2$ par injection.

6. Utilisation selon la revendication 5 caractérisée en ce que l'IL2 est comprise dans une composition pharmaceutique qui permet l'administration de l'IL2 à la dose de 15 x $10^6$ U/M$^2$ par jour.

7. Utilisation selon la revendication 6 caractérisée en ce que l'IL2 est comprise dans une composition pharmaceutique qui permet l'administration de l'IL2 par cycle de 5 jours consécutifs.

8. Utilisation selon la revendication 7 caractérisée en ce que l'IL2 est comprise dans une composition pharmaceutique qui permet l'administration de l'IL2 de façon répétée pendant au moins deux cycles non consécutifs.

**Patentansprüche**

1. Verwendung eines Polypeptids mit einer Aktivität des humanen Interleukin 2 zur Herstellung einer pharmazeutischen Zusammensetzung für die Behandlung von primitivem Pleura-Krebs durch intrapleurale Verabreichung.

2. Verwendung gemäß Anspruch 1, dadurch gekennzeichnet, daß der Pleura-Krebs ein Mesotheliom ist.

3. Verwendung gemäß Anspruch 2, dadurch gekennzeichnet, daß das Polypeptid mit einer Aktivität des humanen IL2 eine reine IL2-Rekombinante ist.

4. Verwendung gemäß Anspruch 3, dadurch gekennzeichnet, daß das IL2 eine nicht-glykosylierte IL2-Rekombinante in reduzierter Form ist.

5. Verwendung gemäß Anspruch 3 oder 4, dadurch gekennzeichnet, daß das IL2 in einer pharmazeutischen Zusammensetzung enthalten ist, die die Verabreichung von IL2 auf intrapleuralem Weg in einer Dosis von 2 bis 25 x $10^6$ E/M$^2$ durch Injektion erlaubt.

6. Verwendung gemäß Anspruch 5, dadurch gekennzeichnet, daß das IL2 in einer pharmazeutischen Zusammensetzung enthalten ist, die die Verabreichung von IL2 in einer Dosis von 15 x $10^6$ E/M$^2$ je Tag erlaubt.

7. Verwendung gemäß Anspruch 6, dadurch gekennzeichnet, daß das IL2 in einer pharmazeutischen Zusammensetzung enthalten ist, die die Verabreichung von IL2 in einem Zyklus von 5 aufeinanderfolgenden Tagen erlaubt.

8. Verwendung gemäß Anspruch 7, dadurch gekennzeichnet, daß das IL2 in einer pharmazeutischen Zusammensetzung enthalten ist, die die wiederholte Verabreichung von IL2 während zumindest 2 nicht aufeinanderfolgenden Zyklen erlaubt.

**Claims**

1. The use of a polypeptide having the activity of human interleukin 2 for the preparation of a pharmaceutical composition intended for the treatment of primary cancers of the pleura, by administration by intrapleural route.

2. Use according to claim 1 characterized in that the cancer of the pleura is a mesothelioma.

3. Use according to claim 2 characterized in that the polypeptide having the activity of human IL2 is a pure recombinant IL2.

4. Use according to claim 3 characterized in that the IL2 is a non-glycosylated recombinant IL2 in reduced form.

5. Use according to claim 3 or 4 characterized in that the IL2 is contained in a pharmaceutical composition which allows the administration of the IL2 by intrapleural route at a dose of 2 to 25 x $10^6$ U/M$^2$ by injection.

6. Use according to claim 5 characterized in that the IL2 is contained in a pharmaceutical composition which allows the administration of the IL2 at a dose of 15 x $10^6$ U/M$^2$ per day.

7. Use according to claim 6 characterized in that the IL2 is contained in a pharmaceutical composition that allows the administration of the IL2 in a cycle of 5 consecutive days.

8. Use according to claim 7 characterized in that the IL2 is contained in a pharmaceutical composition which allows the administration of the IL2 in a repeated manner for at least two non-consecutive cycles.